# EUROPÄISCHE PATENTSCHRIFT

(11) **EP 4 427 016 B1**
(45) Veröffentlichungstag und Bekanntmachung des Hinweises auf die Patenterteilung: **26.02.2025**
(21) Anmeldenummer: 23812914.2
(22) Anmeldetag: 23.11.2023
(51) Int. Cl.: G01N 1/28, B02C 23/06, G01N 23/2202, B02C 17/20, G01N 23/2005, C04B 40/00, C04B 103/52

(54) **MAHLHILFSTABLETTE MIT INTEGRIERTEM STANDARD**
GRINDING AID TABLET HAVING AN INTEGRATED STANDARD
TABLETTE D'AIDE AU BROYAGE AYANT UNE NORME INTÉGRÉE

(30) Priorität: 02.12.2022 DE 102022132071; 02.12.2022 LU 103044
(43) Veröffentlichungstag der Anmeldung: 11.09.2024
(73) Patentinhaber: thyssenkrupp Polysius GmbH, 59269 Beckum (DE); thyssenkrupp AG, 45143 Essen (DE)
(72) Erfinder: ENDERS, Michael, 48143 Münster (DE); HOLZER, Jasmin, 59320 Ennigerloh (DE)
(74) Vertreter: thyssenkrupp Intellectual Property GmbH
(86) Internationale Anmeldenummer: PCT/EP2023/082821
(87) Internationale Veröffentlichungsnummer: WO 2024/115266

(56) Entgegenhaltungen:
- CN-A- 106 220 032
- DE-B3- 102020 201 877

## Beschreibung

Die Erfindung betrifft eine Mahlhilfstablette mit einer integrierten kristallinen Substanz als Standard zur quantitativen röntgendiffraktometrischen Bestimmung amorpher Bestandteile, beispielsweise für die Klinker- und Zementherstellung.

Proben, beispielsweise in der Zementindustrie, der Aluminiumindustrie oder der Stahlherstellung, werden heutzutage oft, teilweise automatisiert zur Prozesssteuerung, mittels röntgendiffraktometrischer Verfahren quantitativ analysiert. Zur quantitativen Auswertung erfolgt eine Rietveld-Anpassung, wodurch eine quantitative Bestimmung der Phasen zueinander ermöglicht wird. Insbesondere die Verwendung von Flächendetektoren ermöglichen hierbei für eine Prozesssteuerung sinnvoll kurze Messzeiten bei einer quantitativen Analyse bis zu 0,1 % Genauigkeit der einzelnen Phasenanteile. Problematisch sind hierbei die Quantifizierungen amorpher Verbindungen, die regelmäßig daher über eine sogenannte Pseudostruktur angepasst werden.

Mahlhilfsmittel, insbesondere Mahlhilfstabletten, haben drei primäre Aufgaben. Zum einen unterstützen die Mahlhilfsmittel die Zerkleinerungswirkung, wodurch beim Mahlen eine bessere Homogenisierung der Probe erreicht werden kann. Als zweites soll durch die Mahlhilfsmittel eine automatische Reinigung der Mahlaggregate erleichtert werden. Dadurch kann in insbesondere in einer rein automatisierten Probenherstellung eine Kontamination zwischen den aufeinanderfolgenden Proben minimiert werden, indem ein vollständiger Austrag erzielt wird. Diese Bestandteile der Mahlhilfstablette sorgen dann auch als dritten Effekt für eine Stabilisierung der hergestellten Presstablette der Probe, um eine gute Untersuchbarkeit und Handhabbarkeit zu gewährleisten. Mahlhilfsmittel werden daher üblicherweise in der Größenordnung von ungefähr 10 Gew.-% zugegeben.

Die Verwendung der Mahlhilfsmittel in Form der Mahlhilfstablette ermöglicht eine gute, insbesondere automatisierte, Verarbeitung, eine sehr einfache und zuverlässige Dosierung und vermeidet Verunreinigungsprobleme.

Aus der DE 10 2013 106 998 A1 ist ein Verfahren sowie eine Vorrichtung zur Herstellung einer Tablette bekannt.

Aus der DE 10 2020 201 877 B3 ist eine Zusammensetzung einer Mahlhilfstablette bekannt.

Die Stoffe der Mahlhilfsmittel sind üblicherweise amorph und tragen somit zu einem meist ohnehin vorhandenen amorphen Untergrund bei. Dieses erschwert die Quantifizierung amorpher Bestandteile in einer Probe.

Des Weiteren gibt es an eine Mahlhilfstablette neben der bereits genannten drei zu erfüllenden Anforderung natürlich auch die Anforderung, dass die Mahlhilfstablette an sich stabil sein muss, um eine sichere und insbesondere automatisierte Verarbeitung zu ermöglichen.

Aufgabe der Erfindung ist es, möglichst alle Phasen der Probe quantitativ bestimmen zu können.

Gelöst wird diese Aufgabe durch das Mahlhilfsmittel mit den in Anspruch 1 angegebenen Merkmalen sowie durch das Verfahren mit den in Anspruch 8 angegebenen Merkmalen. Vorteilhafte Weiterbildungen ergeben sich aus den Unteransprüchen, der nachfolgenden Beschreibung.

Das erfindungsgemäße Mahlhilfsmittel dient dazu, eine Probe insbesondere automatisch, besser vermahlen zu können und eine stabile Röntgenuntersuchungstablette zur anschließenden Untersuchung mittels Röntgenbeugung herzustellen. Das Mahlhilfsmittel hat dabei die Funktion, ein Vermahlen und damit eine gute Homogenisierung der Probe zu unterstützen, die vollständige Entfernung der Probe aus den Gerätschaften zu unterstützen und anschließend der gepressten Röntgenuntersuchungstablette Stabilität zu verleihen. Nun und erfindungsgemäß kommt zusätzlich die Aufgabe hinzu, einen internen Röntgenstandard bereitzustellen, damit eine quantitative Analyse zuverlässig möglich ist.

Das Mahlhilfsmittel weist erfindungsgemäß die folgende Zusammensetzung auf:
A 15 % bis 35 % Cellulose oder Cellulosederivat,
B 40 % bis 60 % Naturharz,
C 0 % bis 5 % Hilfs- und Beschichtungsstoffe,
D 15 % bis 25 % kristallines anorganisches Material als Röntgenstandard.

Die Summe der der Komponenten A, B, C und D ergibt 100 %. Alle Angaben sind hierbei und im Folgenden in Gew.-%.

Cellulosederivate (A) sind chemische Derivate von Cellulose und werden häufig als Bindemittel zum Beispiel in pharmazeutischen und kosmetischen Produkten eingesetzt. Cellulosederivate werden zum Beispiel durch Methylierung (Methylcellulose), Ethylierung, Hydroxypropylierung, Sulfonierung, Nitrierung (Cellulosenitrat), Acetylierung (Celluloseacetat), Oxidation, Xanthogenierung, Quervernetzung, oder Copolymerisation durch Pfropfen hergestellt. Es gibt zahlreiche weitere Modifikationen.

Naturharz (B) trägt insbesondere durch eine Klebrigkeit zur Stabilisierung einer Probentablette bei.

Hilfs- und Beschichtungsstoffe (C) dienen zum Beispiel dazu, die Oberfläche der Mahlhilfetablette zu schützen, Schütteigenschaften zu verbessern und dergleichen. Derartige Beschichtungen und Beschichtungsverfahren sind dem Fachmann geläufig. Aufgrund des geringen Anteils haben diese Stoffe kaum, optimal keine Auswirkung beim Mahlprozess sowie bei der anschließenden Untersuchung des vermahlenen Materials.

Somit unterscheidet sich die Zusammensetzung bezüglich des zusätzlichen Bestandteils D von herkömmlichen Mahlhilfsmitteln, wie beispielsweise aus der DE 10 2020 201 877 B3 bekannt. Durch den Zusatz an kristallinem anorganischen Material wird jedoch insgesamt eine größere Menge an Mahlhilfsmittel benötigt. Die veränderte Zusammensetzung wiederum bewirkt, dass auch insbesondere das Verhältnis zwischen der Komponente A und der Komponente B verändert ist. Würde man den aus dem Stand der Technik bekannten höheren Anteil an Cellulose verwenden, so ist die anschließend erzeugte Röntgenuntersuchungstablette weniger stabil, und neigt zu Brechen durch Lamination. Das einfache Hinzufügen eines kristallinen anorganischen Materials ohne Anpassung der weiteren Rezeptur ist daher nicht möglich.

Daher ist es auch vorteilhaft, das kristalline anorganische Material bereits dem Mahlhilfsmittel zuzugeben. Zum einen wird bei der anschließenden Verwendung der zweite Dosierschritt für eine getrennte Zugabe eines internen Standards in Form eines kristallinen anorganischen Materials überflüssig. Zum anderen ist dadurch auch das Verhältnis zwischen den weiteren Komponenten des Mahlhilfsmittels und dem kristallinen anorganischen Material konstant und unabhängig, beispielsweise von Dosier- oder Wägefehlern. Die Zusammensetzung der weiteren Komponenten des Mahlhilfsmittels kann daher exakt auf das voreingestellte Verhältnis zwischen den weiteren Komponenten des Mahlhilfsmittels und dem kristallinen anorganischen Material eingestellt werden.

Neben Röntgendiffraktion können auch andere Untersuchungen an der Röntgenuntersuchungstablette vorgenommen werden, entweder zeitgleich oder zeitversetzt. Beispielswiese kann mittels Röntgenfluoreszenz eine Zusammensetzung untersucht werden. Ebenso kann optional eine Untersuchung mittel Infrarotspektroskopie erfolgen. Dieses ist ein weiterer Vorteil der Röntgenuntersuchungstablette, es können mehrere zerstörungsfreie Untersuchungen an der gleichen Röntgenuntersuchungstablette durchgeführt werden.

In einer weiteren Ausführungsform der Erfindung weisen Komponente A und Komponente B ein Verhältnis von 25 zu 75 bis 35 zu 65 zueinander auf. Hierdurch wird eine optimale Festigkeit bei minimaler Brüchigkeit der Röntgenuntersuchungstablette erreicht. Gleichzeitig wird auch eine gute Selbstreinigung der Mühle erreicht. Dieses Verhältnis unterscheidet sich deutlich, von einem Mahlhilfsmittel ohne kristallines anorganisches Material, wie beispielsweise aus der DE 10 2020 201 877 B3 bekannt. Dieses Verhältnis hat sich bei dem notwendigen hohen Anteil an kristallinem anorganischen Material als optimal herausgestellt.

In einer weiteren Ausführungsform der Erfindung weist die Komponente D einen oder mehrere Stoffe aus der Liste umfassend Titandioxid, insbesondere Rutil, Aluminiumoxid, insbesondere Korund, Siliziumdioxid, insbesondere Quarz, Siliziumcarbid, Silizium, Hämatit, Magnetit, auf. Bevorzugt ist der ausgewählte Stoff nicht auch Bestandteil der Probe. Typische Anwendungsbereiche der automatisierten Röntgendiffraktometrie zur Prozesssteuerung finden sich heutzutage zum Beispiel in der Zementindustrie, der Eisen- und Stahlherstellung, insbesondere auch im Bereich der Schlacken, der Aluminiumherstellung oder der Titandioxidherstellung. Der Fachmann wird ein geeignetes kristallines anorganisches Material auswählen, welches im Diffraktogramm den geringsten Überlapp zu den Peaks des kristallinen Materials der Probe aufweist.

In einer weiteren Ausführungsform der Erfindung beträgt der Anteil der Komponente A zwischen 28 % und 32 %, bevorzugt 30 %.

In einer weiteren Ausführungsform der Erfindung enthält das Naturharz der Komponente B die folgenden Komponenten:
a 3 bis 20 % 2,4-bis(1-Phenylethyl)phenol,
b 1 bis 12 % Dehydroabietinsäure
c 1 bis 8 % 1,2-bis(4-Methoxyphenyl)-1-propen
d 1 bis 7 % ((1R, 4aR, bR, 7R, 10aR)-1,4a,7-Trimethyl-7-vinyl-1,2,3,4,4a,4b,6,7,8,9,10,10a-dodecahydrophenanthren-1-yl) methanol
e 1 bis 7 % (1R, 4aR, bR, 7R, 10aR)-1,4a,7-Trimethyl-7-vinyl-1,2,3,4,4a,4b,6,7,8,9,10,10a-dodecahydrophenanthren-1-carbaldehyd.

Die Summe aller Komponenten des Naturharzes ergibt 100%%.

Eine vollständige Analyse aller Komponenten ist naturgemäß nicht möglich und gerade bei den Substanzen mit sehr geringem Anteil kann es zu deutlichen Schwankungen kommen. Weiter weist das Naturharz regionale und jährliche Schwankungen auf, daher ist eine exakte Zusammensetzung nicht angebbar. Schwankungen kommen beispielsweise durch zeitliche, regionale oder klimatische Unterschiede beim Pflanzenwachstum zustande.

Zur Untersuchung des Naturharzes wurden zum einen 1D-NMR und 2D-NMR verwendet. Zum anderen wurde eine Pyrolyse bei 450 °C für 10 s durchgeführt und die Produkte anschließend über eine GC-Säule HP-5 ms mit einem Temperaturprogramm 45 - 240 °C mit 4 K/min und anschließend 240 - 300 °C mit 40 K/min sowie 15 min Haltezeitbei mit Helium als Trägergas mit einer Strömungsgeschwindigkeit von 1 ml / min bei einem Split von 50 : 1. Die getrennten Stoffe wurden mittels Massenspektroskopie identifiziert, wobei ein Scan von 10 - 600 g/mol durchgeführt wurde.

In einer weiteren Ausführungsform der Erfindung enthält das Naturharz der Komponente B zusätzlich die folgenden Komponenten:

| | |
|---|---|
| f | 0,5 bis 6 % Longifolen |
| g | 0,5 bis 6 % Abietinsäure |
| h | 0,5 bis 5 % Caryophyllenoxid |

In einer weiteren Ausführungsform der Erfindung enthält das Naturharz der Komponente B zusätzlich die folgenden Komponenten:

| | |
|---|---|
| i | 2 bis 25 % 9-Ethyl-1,2,3,4,5,6,7,8-octahydroanthracen |
| j | 2 bis 25 % N-(2-Ethylphenyl) phenylacetamid |

In einer weiteren Ausführungsform der Erfindung enthält das Naturharz der Komponente B die folgende Komponente:

| | |
|---|---|
| b | 3 bis 8 % Dehydroabietinsäure |

In einer weiteren Ausführungsform der Erfindung enthält das Naturharz der Komponente B die folgenden Komponenten:

| | |
|---|---|
| d | 2 bis 5,5 % ((1R, 4aR, bR, 7R, 10aR)-1,4a,7-Trimethyl-7-vinyl-1,2,3,4,4a,4b,6,7,8,9,10,10a-dodecahydrophenanthren-1-yl) methanol |
| e | 2 bis 5,5 % (1R, 4aR, bR, 7R, 10aR)-1,4a,7-Trimethyl-7-vinyl-1,2,3,4,4a,4b,6,7,8,9,10,10a-dodecahydrophenanthren-1-carbaldehyd |

In einer weiteren Ausführungsform der Erfindung weist das Mahlhilfsmittel eine Beschichtung aus PMMA auf. Hierdurch wird die Handhabung erleichtert und gleichzeitig ein Materialverlust bei der Handhabung, beispielsweise durch Abrieb oder Abbruch verringert.

In einer weiteren Ausführungsform der Erfindung ist das Mahlhilfsmittel eine Mahlhilfstablette mit einer Masse von 0,2 bis 0,4 g. Bei üblichen Probengrößen um 10 g (meist 5 g bis 20 g) als geeignet erwiesen, um eine genaue Dosierung und gleichzeitig keine zu hohe Anzahl an benötigten Mahlhilfstablette und damit einen zu hohen Dosierungsaufwand zu haben.

In einem weiteren Aspekt betrifft die Erfindung ein Verfahren zur quantitativen Bestimmung der Phasenanteile einer Probe mittels Röntgendiffraktometrie. Das Verfahren weist die folgenden Schritte auf:
a) Einwaage der Probe,
b) Zudosieren des Mahlhilfsmittels nach einem der vorstehenden Ansprüche,
c) Vermahlen,
d) Pressen einer Röntgenuntersuchungstablette,
e) Erfassen des Röntgendiffraktogramms,
f) Rietveld-Anpassung des Röntgendiffraktogramms.

Erfindungsgemäß zeichnet sich das Verfahren dadurch aus, dass
g) über das Verhältnis der der kristallinen Phase der Probe im Verhältnis zur kristallinen anorganischen Materials des Mahlhilfsmittels der quantitative Anteil der amorphen Phase der Probe bestimmt wird.

Die Einwaage in Schritt a) kann entweder exakt oder ungefähr genau erfolgen. Bei einer exakten Einwaage wird eine Vorgabe von beispielsweise 10,00 g vorgegeben und exakt diese Masse an Probe abgewogen. Alternativ kann bei der ungefähr genauen Einwaage eine Vorgabe von ungefähr 10 g, beispielsweise zwischen 8 g und 12 g erfolgen, wobei dann bei der exakten Bereitstellung die genaue Masse, beispielsweise 11,11 g gemessen wird. Beide Verfahren haben ihre Vorteile und Nachteile.

Das Zudosieren in Schritt b) erfolgt besonders einfach, wenn Mahlhilfsmittel als Mahlhilfstablette vorliegt, beispielsweise in einer Masse von 250 mg je Mahlhilfstablette. Werden zum Beispiel 10 g Probe vorgegeben werden beispielsweise 4 Mahlhilfstabletten mit insgesamt 1 g zudosiert. Die Zahl vier ist vergleichsweise einfach zu zählen und dosieren, und der Messfehler bleibt dadurch minimal.

Anschließend erfolgt das Vermahlen in Schritt c) und das Pressen einer Röntgenuntersuchungstablette in Schritt d).

Die in Schritt d) erzeugte Röntgenuntersuchungstablette wird in Schritt e) mittels Röntenbeugung untersucht, beispielsweise mittels einer Cu K_{α}-Quelle und einem 2D-Flächendetektor. Wesentlich ist, dass das in Schritt e) erfasste Röntgendiffraktogramm eine ausreichende Qualität für die in Schritt f) folgende Rietveld-Anpassung aufweist.

Die Basis der Rietfeld-Methode zur Anpassung ist es, die bekannten Phasen vorzugeben und deren bekannten Reflexe (Position und Intensitätsverhältnis). Nun werden die für die Untersuchung wesentlichen Parameter variiert, insbesondere das Verhältnis der Phasen zueinander, aber auch beispielsweise die Partikelgröße (was zu einer Reflexverbreiterung führen kann). Letzteres kann insbesondere geeignet sein, um beispielsweise einen amorphen oder pseudoamorphen Anteil mit zu kalkulieren. Es erfolgt dann ein Abgleich zwischen dem simulierten Diffraktogramm und dem in Schritt e) erfassten Diffraktogramm, wobei das Optimum nach der Methode der kleinsten Quadrate ermittelt wird. Hierdurch kann das Verhältnis der unterschiedlichen kristallinen Phasen zueinander sehr gut ermittelt werden, sodass eine quantitative Untersuchung der Phasen möglich ist. Beispielsweise kann in einem Klinker auf diese Weise das Verhältnis von Alit (C₃S) zu Belit (C₂S) bestimmt werden.

Da der amorphe Anteil schwierig zu bestimmen und auch von den amorphen Bestandteilen des Mahlhilfsmittels überlagert wird, erfolgt die quantitative Ermittlung des amorphen Anteils aus dem Verhältnis des Anteils der kristallinen Probe zum Anteil des kristallinen anorganischen Materials als Röntgenstandard. Dieses soll im Folgenden beispielhaft erläutert werden.

Beispielsweise werden 10 g Probe mit 1 g Mahlhilfsmittel verarbeitet, wobei das Mahlhilfsmittel 20 % Rutil, also 0,2 g Rutil aufweist. Nimmt man rein hypothetisch und vereinfachend an, dass die Probe zu gleichen Teilen Alit und Belit enthält, so müsste sich bei einer vollständig kristallinen Probe ein Verhältnis von Alit : Belit : Rutil von 25 : 25 : 1 ergeben. Ergibt sich nach der Rietveld-Analyse ein Verhältnis von 12,5 : 12,5 :1, so kann nur die Hälfte der Probe kristallin sein. Somit ergibt sich, dass in diesem sehr einfachen hypothetischen Fall der quantitative Anteil der amorphen Phase in der Probe 50 % betragen würde.

Dieses ist derart einfach, da durch die Verwendung eines Mahlhilfsmittels mit einem internen Standard in einfacher Weise klare Beziehungen auch unter Berücksichtigung der amorphen Bestandteile des Mahlhilfsmittels möglich sind.

In einer weiteren Ausführungsform der Erfindung erfolgt in Schritt b) eine Zudosierung von 0,07 bis 0,12 g Mahlhilfsmittel je 1 g Probe. Die Zudosierung ist durch das zusätzliche kristalline anorganische Material tendenziell höher, darf aber nicht zu hoch sein, um dennoch gute Eigenschaften der Röntgenuntersuchungstablette zu gewährleisten und auch nicht zu stark das Röntgendiffraktogramm der Probe zu überlagern.

In einer weiteren Ausführungsform der Erfindung wird für eine Zement- oder Zementklinkerprobe Rutil als kristallines anorganisches Material verwendet.

In einer weiteren Ausführungsform der Erfindung wird für eine Titandioxidprobe Korund, Silizium oder Siliziumcarbid als kristallines anorganisches Material verwendet.

## Patentansprüche

1. Mahlhilfsmittel, wobei das Mahlhilfsmittel die folgende Zusammensetzung aufweist:
A 15 % bis 35 % Cellulose oder Cellulosederivat,
B 40 % bis 60 % Naturharz,
C 0 % bis 5 % Hilfs- und Beschichtungsstoffe,
D 15 % bis 25 % kristallines anorganisches Material als Röntgenstandard,
wobei die Summe der der Komponenten A, B, C und D 100 % ergibt, wobei alle Angaben in Gew.-% sind.

2. Mahlhilfsmittel nach Anspruch 1, **dadurch gekennzeichnet, dass** Komponente A und Komponente B ein Verhältnis von 25 zu 75 bis 35 zu 65 zueinander aufweisen.

3. Mahlhilfsmittel nach einem der vorstehenden Ansprüche, **dadurch gekennzeichnet, dass** die Komponente D einen oder mehrere Stoffe aufweist aus der Liste umfassend Titandioxid, insbesondere Rutil, Aluminiumoxid, insbesondere Korund, Siliziumdioxid, insbesondere Quarz, Siliziumcarbid, Silizium, Hämatit, Magnetit.

4. Mahlhilfsmittel nach einem der vorstehenden Ansprüche, **dadurch gekennzeichnet, dass** der Anteil der Komponente A zwischen 28 % und 32 % beträgt.

5. Mahlhilfsmittel nach einem der vorstehenden Ansprüche, **dadurch gekennzeichnet, dass** das Naturharz der Komponente B die folgenden Komponenten enthält:
a 3 bis 20 % 2,4-bis(1-Phenylethyl)phenol,
b 1 bis 12 % Dehydroabietinsäure
c 1 bis 8 % 1,2-bis(4-Methoxyphenyl)-1-propen
d 1 bis 7 % ((1R, 4aR, bR, 7R, 10aR)-1,4a,7-Trimethyl-7-vinyl-1,2,3,4,4a,4b,6,7,8,9,10,10a-dodecahydrophenanthren-1-yl) methanol
e 1 bis 7 % (1R, 4aR, bR, 7R, 10aR)-1,4a,7-Trimethyl-7-vinyl-1,2,3,4,4a,4b,6,7,8,9,10,10a-dodecahydrophenanthren-1-carbaldehyd
wobei die Summe aller Komponenten des Naturharzes 100 % ergibt.

6. Mahlhilfsmittel nach einem der vorstehenden Ansprüche, **dadurch gekennzeichnet, dass** das Mahlhilfsmittel eine Beschichtung aus PMMA aufweist.

7. Mahlhilfsmittel nach einem der vorstehenden Ansprüche, **dadurch gekennzeichnet, dass** das Mahlhilfsmittel eine Mahlhilfstablette mit einer Masse von 0,2 bis 0,4 g ist.

8. Verfahren zur quantitativen Bestimmung der Phasenanteile einer Probe mittels Röntgendiffraktometrie, wobei das Verfahren die folgenden Schritte aufweist:
a) Einwaage der Probe,
b) Zudosieren des Mahlhilfsmittels nach einem der vorstehenden Ansprüche,
c) Vermahlen,
d) Pressen einer Röntgenuntersuchungstablette,
e) Erfassen des Röntgendiffraktogramms,
f) Rietveld-Anpassung des Röntgendiffraktogramms,
**dadurch gekennzeichnet, dass**
g) über das Verhältnis der der kristallinen Phase der Probe im Verhältnis zur kristallinen anorganischen Materials des Mahlhilfsmittels der quantitative Anteil der amorphen Phase der Probe bestimmt wird.

9. Verfahren nach Anspruch 8, **dadurch gekennzeichnet, dass** in Schritt b) eine Zudosierung von 0,07 bis 0,12 g Mahlhilfsmittel je 1 g Probe erfolgt.

10. Verfahren nach einem der Ansprüche 8 bis 9, **dadurch gekennzeichnet, dass** für eine Zement- oder Zementklinkerprobe Rutil als kristallines anorganisches Material verwendet wird.

11. Verfahren nach einem der Ansprüche 8 bis 10, **dadurch gekennzeichnet, dass** für eine Titandioxidprobe Korund, Silizium oder Siliziumcarbid als kristallines anorganisches Material verwendet wird.

## Claims

1. A grinding aid, wherein the grinding aid has the following composition:
A 15 % to 35 % cellulose or cellulose derivative,
B 40 % to 60 % natural resin,
C 0 % to 5 % auxiliary and coating materials,
D 15 % to 25 % crystalline inorganic material as X-ray standard,
where the sum of components A, B, C and D is 100 %, all figures being in % by weight.

2. A grinding aid according to claim 1, **characterised in that** component A and component B have a ratio of 25 to 75 to 35 to 65 to each other.

3. Grinding aid according to one of the preceding claims, **characterised in that** component D comprises one or more substances from the list comprising titanium dioxide, in particular rutile, aluminium oxide, in particular corundum, silicon dioxide, in particular quartz, silicon carbide, silicon, hematite, magnetite.

4. Grinding aid according to one of the preceding claims, **characterised in that** the proportion of component A is between 28% and 32%.

5. Grinding aid according to one of the preceding claims, **characterised in that** the natural resin of component B contains the following components:
a 3 to 20 % 2,4-bis(1-phenylethyl)phenol,
b 1 to 12 % dehydroabietic acid
c 1 to 8 % 1,2-bis(4-methoxyphenyl)-1-propene
d 1 to 7 % ((*1R*, *4aR, bR,* 7R, *10aR*)-1,4a,7-trimethyl-7-vinyl-1,2,3,4,4a,4b,6,7,8,9,10,10a-dodecahydrophenanthren-1-yl) methanol
e 1 to 7 % *(1R, 4aR, bR, 7R, 10aR*)-1,4a,7-trimethyl-7-vinyl-1,2,3,4,4a,4b,6,7,8,9,10,10a-dodecahydrophenanthrene-1-carbaldehyde
where the sum of all components of the natural resin is 100 %.

6. Grinding aid according to one of the preceding claims, **characterised in that** the grinding aid has a coating of PMMA.

7. Grinding aid according to one of the preceding claims, **characterised in that** the grinding aid is a grinding aid tablet with a mass of 0.2 to 0.4 g.

8. A method of quantitatively determining the phase fractions of a sample by X-ray diffraction, the method comprising the following steps:
a) Weighing of the sample,
b) Addition of the grinding aid according to one of the preceding claims,
c) Ground,
d) Pressing an X-ray examination tablet,
e) Acquisition of the X-ray diffractogram,
f) Rietveld adjustment of the X-ray diffractogram,
**characterised in that**
g) The quantitative proportion of the amorphous phase of the sample is determined via the ratio of the crystalline phase of the sample in relation to the crystalline inorganic material of the grinding aid.

9. Process according to claim 8, **characterised in that** in step b) an addition of 0.07 to 0.12 g of grinding aid per 1 g of sample takes place.

10. Method according to one of claims 8 to 9, **characterised in that** rutile is used as crystalline inorganic material for a cement or cement clinker sample.

11. Method according to one of claims 8 to 10, **characterised in that** corundum, silicon or silicon carbide is used as crystalline inorganic material for a titanium dioxide sample.

## Revendications

1. Adjuvant de broyage, dans lequel l'adjuvant de broyage a la composition suivante :
A 15 % à 35 % de cellulose ou de dérivé de cellulose,
B 40 % à 60 % de résine naturelle,
C 0 % à 5 % de matériaux auxiliaires et de revêtement,
D 15 % à 25 % de matière inorganique cristalline comme étalon radiographique,
où la somme des composants A, B, C et D est de 100 %, tous les chiffres étant exprimés en % en poids.

2. Adjuvant de broyage selon la revendication 1, **caractérisé par le fait que** le composant A et le composant B ont un rapport de 25 à 75 à 35 à 65 l'un par rapport à l'autre.

3. Aide de broyage selon l'une des revendications précédentes, **caractérisée par le fait que** le composant D comprend une ou plusieurs substances de la liste comprenant le dioxyde de titane, en particulier le rutile, l'oxyde d'aluminium, en particulier le corindon, le dioxyde de silicium, en particulier le quartz, le carbure de silicium, le silicium, l'hématite, la magnétite.

4. Aide au broyage selon l'une des revendications précédentes, **caractérisée par le fait que** la proportion du composant A est comprise entre 28% et 32%.

5. Aide à la rectification selon l'une des revendications précédentes, **caractérisée par le fait que** la résine naturelle du composant B contient les composants suivants :
a 3 à 20 % de 2,4-bis(1-phényléthyl)phénol,
b 1 à 12 % d'acide déhydroabiétique
c 1 à 8 % 1,2-bis(4-méthoxyphényl)-1-propène
d 1 à 7 % *((1R, 4aR, bR,* 7R, *10aR*)-1,4a,7-triméthyl-7-vinyl-1,2,3,4,4a,4b,6,7,8,9,10,10a-dodécahydrophénanthren-1-yl) méthanol
e 1 à 7 % *(1R, 4aR, bR, 7R,* 10aR)-1,4a,7-triméthyl-7-vinyl-1,2,3,4,4a,4b,6,7,8,9,10,10a-dodécahydrophénanthrène-1-carbaldéhyde
où la somme de tous les composants de la résine naturelle est de 100 %.

6. Aide à la mouture selon l'une des revendications précédentes, **caractérisée par le fait que** l'aide à la mouture a un revêtement en PMMA.

7. Adjuvant de broyage selon l'une des revendications précédentes, **caractérisé par le fait que** l'adjuvant de broyage est une pastille d'adjuvant de broyage d'une masse de 0,2 à 0,4 g.

8. Méthode de détermination quantitative des fractions de phase d'un échantillon par diffraction des rayons X, comprenant les étapes suivantes :
a) Pesée de l'échantillon,
b) Ajout de l'adjuvant de broyage selon l'une des revendications précédentes,
c) Le sol,
d) Appuyer sur une tablette d'examen radiologique,
e) Acquisition du diffractogramme de rayons X,
f) Ajustement Rietveld du diffractogramme des rayons X,
se **caractérise par le fait que**
g) La proportion quantitative de la phase amorphe de l'échantillon est déterminée par le rapport entre la phase cristalline de l'échantillon et le matériau inorganique cristallin de l'adjuvant de broyage.

9. Procédé selon la revendication 8, **caractérisé par le fait que** à l'étape b), on ajoute de 0,07 à 0,12 g d'adjuvant de broyage par 1 g d'échantillon.

10. Méthode selon l'une des revendications 8 à 9, **caractérisée par le fait que** rutile est utilisé comme matériau inorganique cristallin pour un échantillon de ciment ou de clinker.

11. Méthode selon l'une des revendications 8 à 10, **caractérisée par** l'utilisation de corindon, silicium ou carbure de silicium comme matériau inorganique cristallin pour un échantillon de dioxyde de titane.
